(19) **Europäisches Patentamt**

**European Patent Office**

**Office européen des brevets**

(11) **EP 0 751 802 B1**

(12) **EUROPEAN PATENT SPECIFICATION**

(45) Date of publication and mention
of the grant of the patent:
**25.10.2000 Bulletin 2000/43**

(21) Application number: **95905926.2**

(22) Date of filing: **13.12.1994**

(51) Int. Cl.[7]: **A61N 1/30**

(86) International application number:
**PCT/US94/14352**

(87) International publication number:
**WO 95/26781 (12.10.1995 Gazette 1995/43)**

(54) **DESCRIPTION TRANSDERMAL DRUG DELIVERY BY ELECTROINCORPORATION OF MICROCARRIERS**

TRANSDERMALE IONTOPHORETISCHE ABGABE VON MEDIKAMENTEN IN MIKROTRÄGERN

ADMINISTRATION TRANSDERMIQUE DE MEDICAMENTS PAR ELECTROINCORPORATION DE MICROVECTEURS

(84) Designated Contracting States:
**AT BE CH DE DK ES FR GB IE IT LI NL SE**

(30) Priority: **30.03.1994 US 219970**
**22.09.1994 US 310647**

(43) Date of publication of application:
**08.01.1997 Bulletin 1997/02**

(73) Proprietor: **Genetronics, Inc.**
**San Diego, CA 92121 (US)**

(72) Inventor: **HOFMANN, Gunter A.**
**San Diego, CA 92109 (US)**

(74) Representative:
**Gates, Marie Christina Esther et al**
**c/o Tomkins & Co.**
**5 Dartmouth Road**
**Dublin 6 (IE)**

(56) References cited:
**WO-A-92/04937**     **US-A- 4 942 883**
**US-A- 5 162 042**     **US-A- 5 318 514**

Printed by Xerox (UK) Business Services
2.16.7 (HRS)/3.6

**Description**

TECHNICAL FIELD

The present invention relates to drug delivery and pertains particularly to an apparatus for the transdermal delivery of drugs and other molecules.

BACKGROUND ART

The concept of transdermal drug delivery as a means of delivering drugs and the like without the physical penetration of the tissue has been around for many years. Transdermal drug delivery has the advantage of being highly precise and consistent, similar to intravenous drip but noninvasive because there is no needle. The first skin patch that delivered controlled amounts of a drug continuously was introduced about ten years ago. The process involves the placement of a patch containing a drug on the surface of the skin so that the drug is absorbed through the skin. The drug is delivered to the bloodstream with therapeutic levels, providing convenient means to administer the drug over prolonged periods. This traditional transdermal drug delivery is a passive system and is not satisfactory for most applications.

Because of the significant barrier properties of the skin, this passive transdermal drug delivery has been limited to drugs that have a highly-potent low daily doses and can readily penetrate the skin. The skin is highly resistant to penetration by most chemicals including drugs. The outer layer, Stratum Corneum (SC) serves as a highly protective barrier against physical, most chemical and bacterial penetration.

The stratum corneum (SC) consists primarily of a thin layer of dead cells bound together by certain fatty (lipid) materials. This presents a major obstacle to the administration of drugs, immunizing agents, and genes transdermally. The stratum corneum (SC) which consists of a thin layer of dead cells with a high electrical resistance which results in high resistance to normal electroporation. This resistance to normal electroporation was recognized by the Weaver et al patent U.S. 5,019,034 entitled "Control of Transport of Molecules Across Tissue Using Electro-poration". Weaver seeks and proposes electoporation as an alternative to the traditional syringe and gun injection of medications. He describes a proposal for using high voltage, short duration electrical pulses on the tissue surface to produce electroporation of the tissue which comprises the walls of the sweat gland ducts to enable drugs and medication to pass through the sweat gland ducts iontophoretically into the tissue. He also proposes the use of synthetic tissue which has electroporation properties.

I have discovered that this layer can be perforated by the administration of short high voltage electrical field pulses, which creates as what may be appropriatedly returned to dielectric breakdown of the stratum corneum forming pores which can allow the passage of molecules. However, there must be some force to move the molecules through the pores into the underlying tissue.

Another patent of interest is that of Grasso U.S. 4,955,378 entitled "Apparatus and Methods for Performing Electrofusion at Specific Anatomical Sites". He discloses a method of fusing biological particles to living tissue, preferably on corneas and in cervical areas. The tissue consists of living cells which are able to completely fuse with the biological particles, or live cells. This patent is not concerned with and does not address or solve the problem of transdermal transport of drugs. immunizing agents, and genes presented by the resistance of the stratum corneum. Also, he does not recognize the need for or suggest any means to force the drugs immunizing agents, or genes into or across or through the tissue surface.

It is desirable that improved methods and apparatus be available for the transdermal delivery of drugs, immunizing agents, and genes.

DISCLOSURE OF INVENTION

It is the primary object of the present invention to provide an improved apparatus for transdermal drug delivery by electroincorporation of microcarriers. The term electroincorporation means the uptake of external materials such as drugs, proteins and antibodies by electroporation. The term "microcarriers" as used herein refers to microscopic particles for use in an apparatus of the invention.

In accordance with the primary aspect of the present invention, there is provided an apparatus including a source of high voltage pulses and electrodes for applying to tissue surface for trans tissue molecular delivery by electroporation characterized by: a quantity of microscopic particles for carrying molecules to be delivered across a tissue surface; a quantity of molecules to be delivered across the tissue surface embodied in said microscopic particles; a fluid medium containing a quantity of said microscopic particles to be contacted with a selected area of the tissue surface; and means for applying an electric field of sufficient amplitude to induce electroporation of said selected area of tissue and to enable transport of the molecules from the microscopic particles into the tissues. Drugs or genes are loaded into microcarriers, the microcarriers are brought into physical contact with the tissue surface and a pulsed electrical field is applied between the microcarriers and the tissue by means of electrodes. This forms pores at the interface of the microcarriers and the tissue, such that the microcarriers which are larger than the pores fuse with the tissue and form a channel through which drugs and genes, which are under pressure, enter through the tissue.

In another aspect of the invention, the microcarriers are smaller than the pores in the stratum corneum (SC), such that the microcarriers which carry molecules of drugs, immunizing agents, and genes, enter through the SC into the tissue where the molecules are diffused.

## BRIEF DESCRIPTION OF DRAWING

The objects, advantages and features of this invention will be more readily appreciated from the following detailed description, when read in conjunction with the accompanying drawing, in which:

Fig. 1 is a perspective view of an apparatus for carrying out the process of the present invention;
Fig. 2 is an enlarged view of the head assembly of the Fig. 1 embodiment;
Fig. 3 is a diagrammatic illustration of a microcarrier loaded with molecules of drugs, immunizing agents or genes;
Fig. 4 is a diagrammatic illustration of multiple microcarriers applied to the surface of the stratum corneum;
Fig. 5 is a diagrammatic illustration of a step of applying electrodes and a pulse electrical field between the microcarriers and skin or stratum corneum;
Fig. 6 is a diagrammatic illustration of the formation of pores at the interface of microcarriers and the stratum corneum;
Fig. 7 is a diagrammatic illustration of the fusion of the microcarriers with the stratum corneum and the passage of drugs or genes through channels in the stratum corneum.
Fig. 8 is a diagrammatic illustration of the formation of pores and the passage of microcarriers through the pores in the stratum corneum; and
Fig. 9 is a diagrammatic illustration of the microcarriers below the stratum corneum and the passage of drugs, immunizing agents or genes from the vesicles into the skin below the stratum corneum;
Fig. 10 is a diagrammatic illustration of a equipotential and field line distribution around electrodes on the surface of the stratum corneum;
Fig. 11 is a diagrammatic illustration of the field lies around a microbubble and through a pore in the stratum corneum; and
Fig. 12 is a diagrammatic illustration like Fig. 11 for that of a solid microcarrier.

## BEST MODES FOR CARRYING OUT THE INVENTION

The present invention takes advantage of dielectric breakdown of the stratum corneum (SC) to transfer drugs and genes contained in vesicles across the SC surface into the underlying tissue and possibly into the blood stream. When desirable, subsequent electroporation may be applied to improve the uptake of drugs, genes, DNA or the like, into cells in the living tissue of humans and other living organism. Various techniques including electroporation is used to load molecules such as drugs and DNA into microparticles (microcarriers) of a size up to several μm diameters. The term microparticle (microcarrier) as used herein means any suitable carrier such as microbubbles, which can be liposomes, erythrocyte ghosts or other vesicles, and solid carriers. The microcarriers are then applied to the SC and electrodes are then applied over the microcarriers. Electrical field pulses are then used to create dielectric breakdown of the stratum corneum or other tissue surface forming passages through which either the drugs and other molecules or the microcarriers and the drugs or other molecules pass into the underlying tissue. The microcarriers, when larger than the pores are fused to the surface of the skin and the electric fields cause dielectric breakdown of the SC and the microbubbles so that the molecules pass from the microbubbles through the pores. When the microcarrier or bubbles are smaller than the pores they pass through the stratum corneum are then broken down and the molecules diffused into the tissue.

Electroporation involves the transient formation of pores in tissue or cell membranes utilizing a short pulse of high-voltage electric fields. Once these pores are formed in the skin or other tissue, molecules can pass the skin or tissue to desired locations in the tissue. When pores are formed in cell membranes, DNA and other molecules can enter the cells through these pores in the cell walls. Thereafter, they stay encapsulated in the cell and the cell walls reseal themselves. The DNA or other gene or drug can then act within the cell to alter the cell properties.

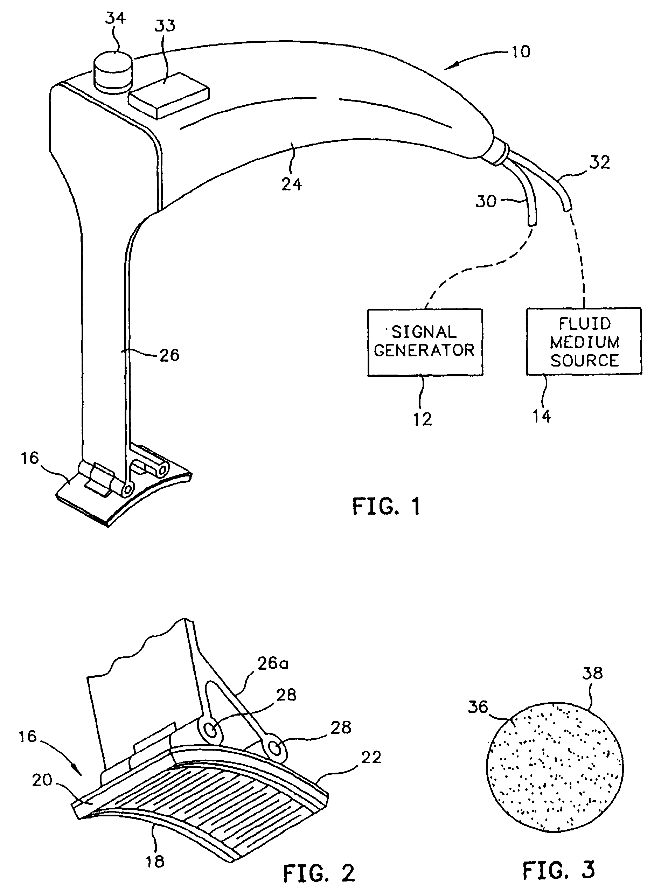

Referring to Fig. 1, an exemplary embodiment of an apparatus which may be utilized in carrying out the process of the present invention, is illustrated. The device comprises a manually positionable applicator designated generally by the numeral 10 which is connected to a signal generator 12 and a fluid medium source 14. The applicator 10 has a head assembly 16 which engages and applies microcarriers with genes, immunizing agents or drugs and electrical pulses to a preselected surface tissue region of a patient. Details of the head assembly are illustrated in Fig. 2.

The head assembly comprises an electrode array 18 which is carried or mounted on a carrier or applicator such as an open pore foam elastomer 20 carried by flexible semirigid or firm dielectric planar support member 22. Adjacent parallel segments of the conductors serve as opposed electrodes for application of the electric field to the tissue surface. The electrodes are preferably small and closely spaced such as about 0.2mm width at about 0.2mm spacing. The applicator may also be a small patch with electrodes on a surface thereof.

The applicator 10 (Fig. 1) further includes a handle portion 24 and an arm portion 26 on which is mounted the head assembly 16. The head assembly 16 is connected to a Y-shaped distal end 26a by means of a pair of pins 28. These pins enable the head to flex and conform to the curvature of the skin surface.

The terminal ends of the conductors 18 and 19 are connected to the signal generator 12 by way or an electrical cable 30. A fluid medium carrying vesicles containing the molecules or drugs is contained within the fluid medium source 14, which may include a suitable motorized pump or pressure source, not shown. The fluid medium source 14 is coupled to the elastomer foam 20 by flexible tube 32 which extends to the applicator 10 to the foam applicator. An actuator button 33 on the handle 24 of the applicator may be depressed to activate a valve (not shown) and deliver a suitable quantity of the fluid medium to the foam elastomer 20. The elastomer 20 provides a sponge-like substrate for holding a predetermined quantity of the fluid medium in contact with the tissue surface. The signal generator 12 generates short voltage pulses which are applied to the electrodes 18 and 19 by pressing a button 34 which actuates a switch to closee the circuit.

The invention can also be carried out by a catheter type apparatus and methods disclosed in U.S patent 5,318,514 wherein an expandable portion of the cather carries electrodes contacts the tissue surface to apply high voltage pulses. This provides a more convenient apparatus for the delivery of drugs and genes across tissue surfaces and membranes such as in body cavities. The present invention was devised to overcome the problem presented by the resistance of the stratum corneum. However, it is applicable to the insertion of molecules such as drugs and genes across other tissue surfaces in body cavities and open wounds. Certain modifications may be necessary to the illustrated apparatus for these other applications.

Referring to Fig. 3, the process is carried out by first encapsulating the drugs or genes 36 which are to be delivered transdermally into microcarriers 38 such as microbubbles as carriers. These microbubbles can be liposomes, erythrocyte ghosts or other vesicles. The microcarriers may also be of a matrix design where the drug or other molecules are encapsulated within the matrix. This would enable the provision of a time release function. The encapsulation of the molecules can be carried out by any one of a number of known processes, including electroporation.

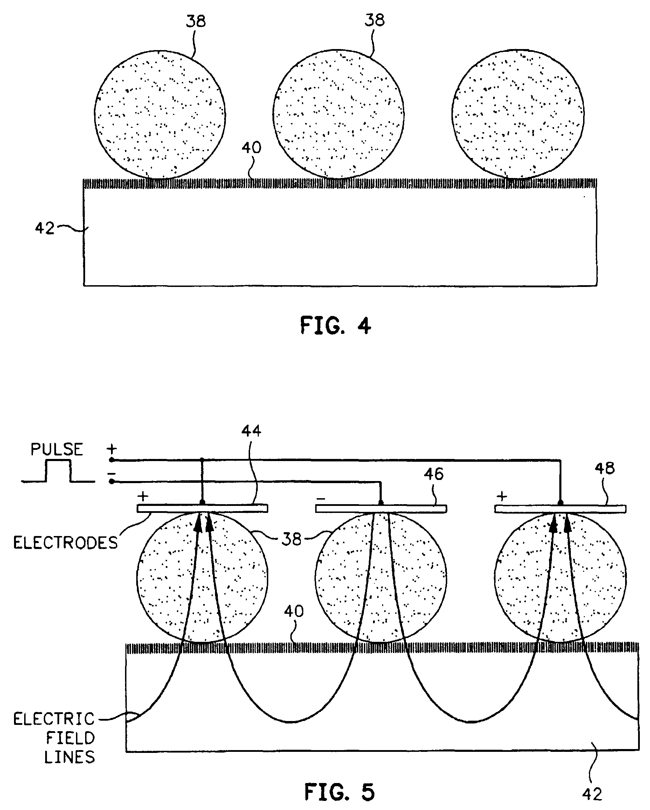

The loaded microbubble, as illustrated in Fig. 4, are then brought into contact with the tissue surface or stratum corneum 40 of a skin layer 42 by suitable means and are positioned between pairs of closely spaced electrodes 44 and 46. This can be carried out by the apparatus of Fig. 1, wherein a fluid carry the microbubbles and applied by the sponge 20 would be positioned between the electrodes 18 on the surface of the applicator. It can also be carried out by a patch having a structure similar to the pad and electrodes a shown in Fig. 2.

Thereafter, a short voltage pulse is applied between the electrodes so that the electric fields of sufficient amplitude are generated to induce dielectric breakdown forming pores in the stratum corneum and in the microbubble and cause the molecules in the microbubble to pass through the pores into the underlying tissues. The microbubble can apply some of the pressure to move the molecules through the pores. As shown in Fig. 5, the electric field is applied so that useful electric field lines are perpendicular to the tissue surface or stratum corneum surface. Typical electrical parameters for the stratum corneum are a field strength of 20 to about 60 kV/cm, which can be generated with moderate voltages of 20 to 120 volts with a pulse length of 10 microseconds (μsec) to 10 milliseconds (msec). This electric field induces a dielectric breakdown in the stratum corneum and in wall of the vesicles or microbubbles. Other tissue surfaces will typically require less field strength.

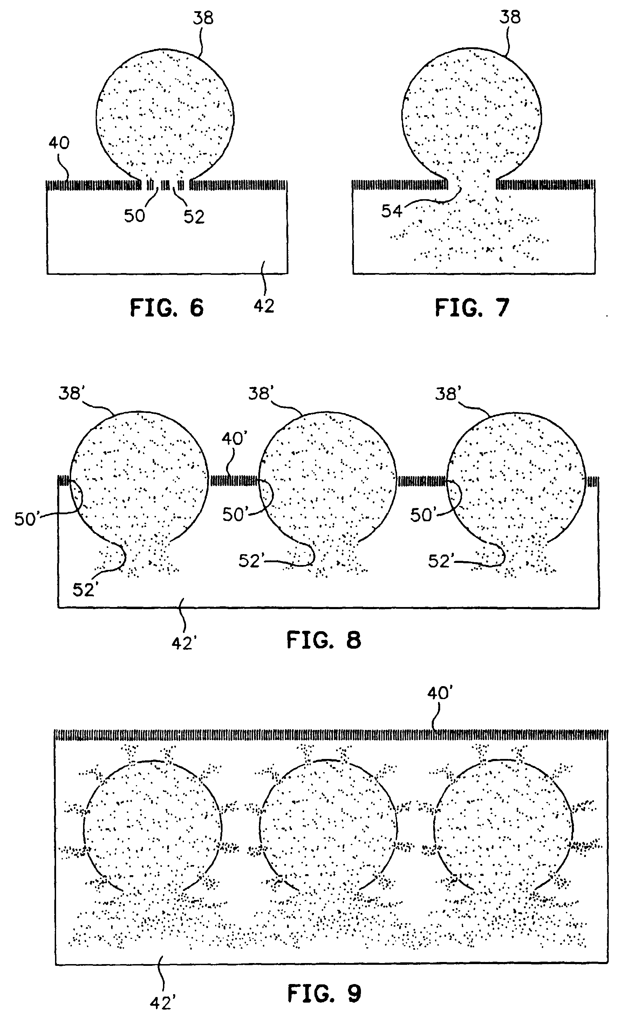

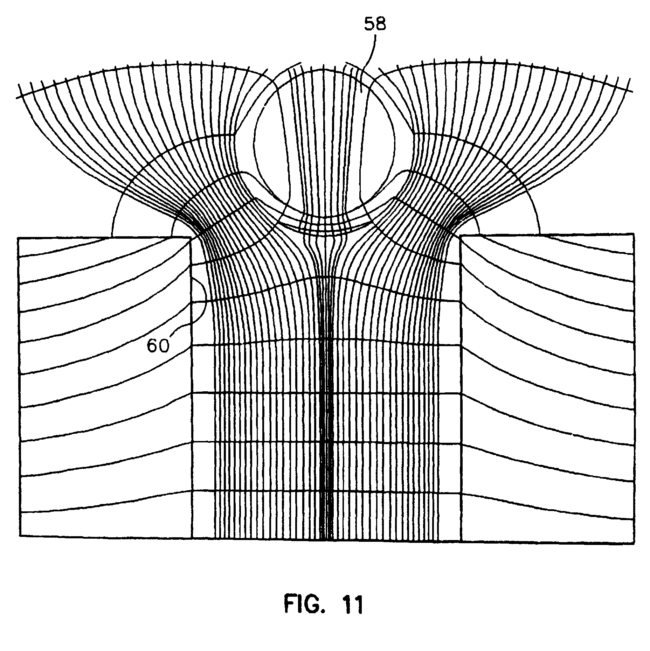

The dielectric breakdown in both the stratum corneum and the microbubbles generate or open pores 50 and 52 as illustrated in Fig. 6. These pores open up further and can join into one lumen and create a channel 54 through which the contents of the microbubbles empty through and into the dermis underlying the stratum corneum as illustrated in Fig. 7. Internal pressure within the microbubbles can act as the driving force to drive the molecules through the channel or channels. Since the stratum corneum consists essentially of dead material, the channel will not close as quickly as it would in a live tissue. This allows the drugs or genes to diffuse trough the surface layer into the underlying skin tissue.

Other forms of a delivery system could be utilized, such as a small system including a patch strapped to the arm or other body part or momentarily connected, containing a rechargeable battery-powered pulse power supply with a reservoir containing microbubbles in suspension with the drug encapsulated. The applicator would have the basic components as the device in Fig. 1 such that by pushing one button, a preselected amount of microbubbles is delivered to the skin between the electrodes. The microbubbles are pressed against the skin for good mechanical contact. Activating another button or switch delivers an electrical pulse to the electrodes which fuse the microbubbles to the stratum corneum. A large number of the microbubbles are then fused to the skin and start pumping or forcing the drug through the stratum corneum.

A special patch having the basic structure of pad 20 can also be applied to the tissue surface. The microbubbles can be contained in the patch Which also contains the electrode structure to create the electric field. The electrode structure can be similar to Fig. 2 and inside the patch. The electrode structure is connected to two electrodes outside the patch so that a pulse generator can be connected momentarily to these outside electrodes to provide a voltage pulse. The patch is preferably provided with an adhesive border to adhere it to the skin or tissue. It is also preferably provided with a protective cover which can be peeled off before adhering the patch to the skin or tissue.

If the drug is to be transported into the cells, a second pulse of appropriate voltage and duration after allowing appropriate diffusion time, is applied to open up pores in the cells. This allows the cells to take up the drug or molecules as in electroporation. The parameters for cell poration in the body are substantially the same as those in solution and will be known to or available to those of skilled in the art. Such parameters are also available in publications available from Genetronics Inc., of San Diego, California.

A drug delivery time profile can be created by mixing different size microbubbles. The flux can then be controlled by the pore size and the number of microbubbles delivered. The apparatus of the present invention could also use iontophoresis as an additional driving force. The iontophoresis takes advantage of ion charges to cause a migration of the ions or molecules through existing passages or pores in the tissue. The combination could use electroporation to open up channels and pores and then use ionphoresis to induce migration of the drugs or genes further into selected tissue.

The present embodiment of the invention has been demonstrated in experiments as follows:

1. Labelled calcein was placed on the skin of a nude mouse.
2. Labelled calcein was placed on the mouse skin, then electroporated.
3. Labelled calcein was encapsulated in liposomes, then placed on the mouse skin.
4. Labelled calcein was encapsulated in liposomes, then placed on the mouse skin and electrofused to the skin.

The results of this limited experiment showed tat the best penetration of the skin into the underlying skin or tissue was seen in Example 4, with the liposome-encapsulated calcein which had been electrofused to the skin.

Referring to Figs. 8 and 9, an alternate embodiment is illustrated wherein like structure is identified by like numbers primed. In this embodiment, microcarriers 38' are selected to be small enough to pass through the pores 50'. At the present time I believe the maximum size to be about 9 μm or slightly larger. The dielectric breakdown in the stratum corneum allow the carriers to pass through open pores 50' as illustrated in Fig. 8. These pores open up and allow the carriers to pass through and into the dermis underlying the stratum corneum as illustrated in Fig. 9. Enzymes within the dermis act to break down walls of the carriers as soon as they enter it forming openings 52' and cause them to release the molecules into the dermis. Since the stratum corneum consists essentially of dead material, the channel will not close as quickly as it would in a live tissue. This allows the carriers containing drugs or genes to pass through the surface layer into the underlying skin tissue where the molecules are diffused into the tissue.

Other forms of a delivery system could be utilized, such as a small system strapped to the arm or other body part or momentarily connected, containing a rechargeable battery-powered pulse power supply with a reservoir containing vesicles in suspension with the drug encapsulated. The applicator would have the basic components as the device in Fig. 1 such that by pushing one button, a preselected amount of vesicles is delivered to the skin between the electrodes. The microcarriers are pressed against the skin for good mechanical contact. Activating another button or switch delivers an electrical pulse to the electrodes which delivers the microcarriers through the stratum corneum.

A special patch can also be applied to the tissue surface. The microcarriers can be contained in the patch which also contains the electrode structure to create the electric field. The electrode structure can be similar to Fig. 2 and inside or on a surface of the patch. The electrode structure is connected to two electrodes outside the patch so that a pulse generator can be connected momentarily to these outside electrodes to provide a voltage pulse. The patch is preferably provided with an adhesive border to adhere it to the skin or tissue. It is also preferably provided with a protective cover which can be peeled off before adhering the patch to the skin or tissue.

If the drug is to be transported into the cells, a second pulse after allowing appropriate diffusion time, is applied to open up pores in the cells. This allows the cells to take up the drug or molecules by electroporation.

A drug delivery time profile can be created by mixing different size microcarriers. The flux can then be controlled by the pore size and the number of vesicles delivered. The apparatus of the present invention could also use iontophoresis as an additional driving force. The iontophoresis takes advantage of ion charges to cause a migration of the ions or molecules through existing passages or pores in the tissue. The combination could use electroincorporation to deliver vesicles through the SC and then use iontophoresis to induce migration of the drugs, immunizing agents, or genes further into selected tissue.

The present embodiment of the invention has been demonstrated in experiments as follows:

1. Labelled calcein was loaded into small liposomes of about 300nm in diameter, as well as large liposomes of 9 μm diameter. These were placed on the skin of hairless mice and electrodes placed on top of the liposomes in order to create electric fields with components perpendicular to the skin. A pulse of about 60 V and 1.2 msec pulse length was applied.

2. Examination by fluorescent microscopy disclosed that calcium was present in the epidermis and dermis after the pulse, not just in the hair follicles but also in between. Further examination by transmission electromicroscopy "TEM" revealed that whole liposomes were present after the pulse below the SC. This indicates that the liposomes which average in size about 300 nm or 9 $\mu$m had crossed the stratum corneum during the pulse.

3. Further study and examination through TEM disclosed that liposomes decomposed and released their contents into the tissue in the dermis. Further tests showed that calcein was entering the blood stream within minutes after the pulse. Further analysis revealed that starting with an amount of calcein on the skin of 25 $\mu$g the amount found in the blood was about 300 ng per ml. Assuming a total amount of blood of about 5 ml, the total amount of calcein in the blood was about 1.5 $\mu$g. This calculates to an efficiency of 1.5 per 25 which equals about 6%.

In plotting this over a period of time, the plot revealed that the concentration of calcein in the blood rose dramatically during the first five minutes, peaking at 15 minutes and dropping off gradually along an almost constant slope at 90 minutes.

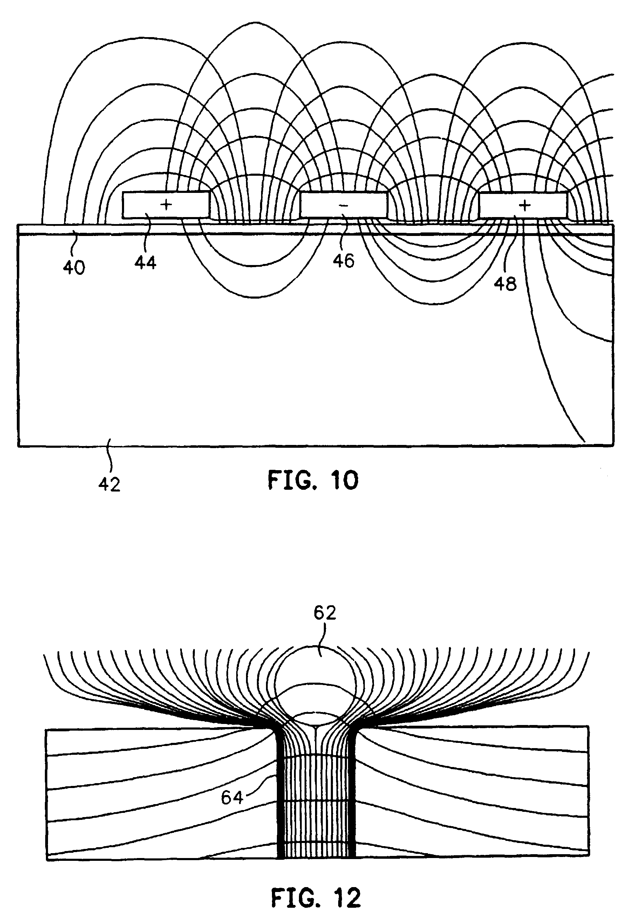

Referring to Fig. 10, an equipotential and electric field line distribution around electrodes of about 0.2 mm in width spaced about 0.2 mm is illustrated. The electrodes 44, 46 and 48 are illustrated on the surface of the SC. The stratum corneum is intact with a high resistivity. The equal potential lines are concentrated in the stratum corneum, leading to a high field strength. The stratum corneum 40 shields the underlying epidermis 42 from the field.

Referring to Fig. 11 a field plot around a liposome 58 of about 300 $\mu$m in diameter at the entrance to a hole 60 in the SC is shown. Charged liposomes will experience a Coulomb force (force on charged particles by an electric field) and can be drawn into the SC and epidermis after break-down of the SC. Uncharged liposomes, such as small liposomes used in my experiments, do not experience a Coulomb force in a homogenous electric field. They are polarized in the electric field and a subjected to a force caused by the inhomogeneous field in the pores of the SC. This "dielectrophoretic" force is proportional to the product of the field strength and the gradient of the field.

Referring to Fig. 12, a similar field plot around a solid particle 62 is illustrated at the entrance to a hole or pore 64 in the SC. This field distribution around a liposome and solid particle in proximity to a pore in the SC is substantially the same.

The following simple model describes the uncharged liposome movement through a pore driving by the electrophoretic force:

1. Dielectric breakdown of SC: $E \geq 20\ kV/cm$

2 Dielectrophoretic force $F_D$ on neutral particles:

$$F_D = \frac{aV}{2}\,\nabla|E|^2$$

$a$ = Polarizability
$V$ = Volume
$E$ = Field Strength

3. Stokes force $F_S$ determines velocity $v$:

$$F_S = 6\pi r v \eta$$

$r$ = radius of particle
$v$ = velocity
$\eta$ = viscosity of medium

$$v = \frac{a}{9\eta}\,r^2\nabla|E|^2$$

4. Pulse duration determines penetration depth $d$:

$$d = VTN$$

$T$ = pulse length
$N$ = number of pulses

5. Example:

2r = 9 um
E = 36kV/cm
3 pulses at 1 msec each

Penetration depth d = 129 um

A more accurate estimate would require knowledge of the shape of the electric field in pores in the SC. Electroincorporation is expected to work well with solid vesicles as well as with vesicles with a membrane.

Dielectrophoresis as well as electrophoresis as a driving force through the SC do not require a vesicle with a membrane. This is different from the electrofusion mechanism where a membrane is essential. It is expected that electroincorporation can be applied to a wide variety of vesicles or microspheres which contain drugs in a matrix. It will be appreciated that the vesicles must be small enough to pass through pores or openings formed in the SC and skin. At the present time I believe this to be about 9 $\mu$m or slightly larger.

The following study has been conducted:

**Chemical Delivered:** Calcein (MW 623)

**Animal Model:** Shaved Mouse

**Analysis:** Fluorescence Microscopy Picture shows tissue to a depth of about 1,500 μm Stratum Corneum at the top.

**Experimental Conditions:**

1. Topical Calcein
2. Topical Calcein plus electroporation
3. Liposome calcein (24 hours incubation)
4. Liposomal calcein plus electrofusion (5 minutes incubation after electroporation)

**Conclusions:**

1. Little, if any, penetration
2. Minor penetration near surface
3. Major penetration into hair shafts, no uptake into the blood
4. Major penetration into tissue between hair shafts, detectable in the blood in less than 15 minutes.

The results of this limited experiment showed that the best penetration of the skin into the underlying skin or tissue was seen in Example 4, with the liposome-encapsulated calcein and electric pulses.

I have illustrated and described my invention by means of specific embodiments, it is to be understood that numerous changes and modifications may be made therein without departing from the scope of the invention as defined in the appended claims.

**Claims**

1. Apparatus (10) including a source of high voltage pulses (12) and electrodes (44,46,48) for applying to tissue surface for trans tissue molecular delivery by electroporation characterized by :

   a quantity of microscopic particles (38) for carrying molecules (36) to be delivered across a tissue surface (40);
   a quantity of molecules (36) to be delivered across the tissue surface (40) embodied in said microscopic particles (38);
   a fluid medium containing a quantity of said microscopic particles (38) to be contacted with a selected area of the tissue surface (40); and
   means (12,44,46,48) for applying an electric field of sufficient amplitude to induce electroporation (50,52,54) of said selected area (40) of tissue and to enable transport of the molecules from the microscopic particles into the tissue (42) having means (12,44,46,48) for applying a field strength of from about 10 to about 60 kV/cm with a pulse length of from 10 μsec to 10 msec.

2. The apparatus of claim 1 wherein said microscopic particles (38) are of a size to inhibit passage through pores (50,52) induced by said poration.

3. The apparatus of claim 1 wherein said microscopic particles (38') are of a size to pass through pores (50',52') induced by said poration.

4. The apparatus of any preceding claim wherein said microscopic particles are of a matrix construction (38') and the molecules (36) are encapsulated within the matrix.

5. The apparatus of any preceding claim wherein the microscopic particles (38) have an electrical charge.

6. The apparatus of any one of claims 1 to 3 or 5 wherein said microscopic particles (38) have a membrane forming microbubbles and the molecules (36) are encapsulated within the microbubbles.

7. The apparatus of any preceding claim wherein said means for applying the electric field comprises a plurality of closely spaced electrodes (44,46) applied to the surface of the tissue (42) and said field is applied as pulses of from 10 to several hundred volts with a pulse length of between 100 μsec to 100 msec.

8. The apparatus of any preceding claim wherein the microscopic particles (38) are constructed such that the molecules (36) are encapsulated within a matrix to allow a time release function.

9. The apparatus of any preceding claim wherein the electrodes (44,46) are parallel strips conductive mounted on the substrate.

10. The apparatus of any preceding claim comprising a substrate for holding the fluid medium containing the microscopic particles, the substrate preferably being sponge-like.

**Patentansprüche**

1. Vorrichtung (10), umfassend eine Quelle von Pulsen hoher Spannung (12) und Elektroden (44, 46, 48) zum Aufbringen auf eine Gewebeoberfläche zur molekularen Abgabe über das Gewebe hinweg durch iontophoretische Abgabe, gekennzeichnet durch:

eine Menge von mikroskopischen Partikeln (38) zum Tragen von Molekülen (36), die über eine Gewebeoberfläche (40) abgegeben werden sollen;

eine Menge von Molekülen (36), die über die Gewebeoberfläche (40) abgegeben werden sollen, die in den mikroskopischen Partikeln (38) enthalten sind;

ein Fluid, das eine Menge der mikroskopischen Partikel (38) enthält, die mit einem ausgewählten Gebiet der Gewebeoberfläche (40) in Kontakt zu bringen sind; und

eine Einrichtung (12, 44, 46, 48) zum Aufbringen eines elektrischen Felds mit ausreichender Amplitude, um eine Elektroporenbildung (50, 52, 54) des ausgewählten Gebiets (40) des Gewebes zu induzieren und um einen Transport der Moleküle von den mikroskopischen Partikeln in das Gewebe (42) zu ermöglichen, wobei eine Feldstärke von etwa 10 bis etwa 60 kV/cm mit einer Pulslänge von 10 µs bis 10 msec durch die Einrichtung (12, 44, 46, 48) aufgebracht wird.

2. Vorrichtung nach Anspruch 1, wobei die mikroskopischen Partikel (38) von einer Größe sind, dass ein Durchgang durch Poren (50, 52), die durch die Porenbildung hervorgerufen wurden, verhindert wird.

3. Vorrichtung nach Anspruch 1, wobei die mikroskopischen Partikel (38') von einer Größe sind, dass sie durch Poren (50', 52'), die durch die Porenbildung gebildet sind, gelangen.

4. Vorrichtung nach einem der vorstehenden Ansprüche, wobei die mikroskopischen Partikel eine Matrixkonstruktion (38') haben und die Moleküle (36) in der Matrix eingebettet sind.

5. Vorrichtung nach einem der vorstehenden Ansprüche, wobei die mikroskopischen Partikel (38) eine elektrische Ladung haben.

6. Vorrichtung nach einem der Ansprüche 1 bis 3 oder 5, wobei die mikroskopischen Partikel (38) eine Membran haben, die Mikroblasen bildet, und die Moleküle (36) in den Mikroblasen eingebettet sind.

7. Vorrichtung nach einem der vorstehenden Ansprüche, wobei die Einrichtung zum Aufbringen des elektrischen Felds eine Vielzahl von nah beabstandeten Elektroden (44, 46) umfasst, die auf die Oberfläche des Gewebes (42) aufgebracht werden, und das Feld als Pulse von 10 bis einigen hundert Volts mit einer Pulslänge zwischen 10 µsec und 100 msec aufgebracht wird.

8. Vorrichtung nach einem der vorstehenden Ansprüche, wobei die mikroskopischen Partikel (38) so konstruiert sind, dass die Moleküle (36) innerhalb einer Matrix eingebettet sind, um eine zeitabhängige Freisetzungsfunktion zu ermögichen.

9. Vorrichtung nach einem der vorstehenden Ansprüche, wobei die Elektroden (44, 46) parallele Streifen sind, die leiten, die auf dem Substrat befestigt sind.

10. Vorrichtung nach einem der vorstehenden Ansprüche, umfassend ein Substrat zum Halten des Fluids, das die mikroskopischen Partikel enthält, wobei das Substrat vorzugsweise schwammartig ist.

**Revendications**

1. Appareil (10) comprenant une source d'impulsions à haute tension (12) et des électrodes (44, 46, 48) destinées à être appliquées à la surface d'un tissu pour l'administration transtissulaire de molécules par électroporation caractérisé par :

une quantité de particules microscopiques (38) pour transporter des molécules (36) à administrer à travers une surface de tissu (40);
une quantité de molécules (36) à administrer à travers la surface de tissu (40) incorporées dans lesdites particules microscopiques (38);
un milieu fluide contenant une certaine quantité desdites particules microscopiques (38) à mettre en contact avec une aire sélectionnée de la surface de tissu (40); et
des moyens (12, 44, 46, 48) pour appliquer un champ électrique d'amplitude suffisante pour induire l'électroporation (50, 52, 54) de ladite aire de tissu sélectionnée (40) et pour permettre le transport des molécules depuis les particules microscopiques jusque dans le tissu (42) ayant des moyens (12, 44, 46, 48) pour appliquer une puissance de champ d'environ 10 à environ 60 kV/cm avec une durée d'impulsion comprise dans la plage allant de 10 µs à 10 ms.

2. Appareil selon la revendication 1, dans lequel lesdites particules microscopiques (38) ont une taille interdisant le passage à travers les pores (50, 52) induits par ladite électroporation.

3. Appareil selon la revendication 1, dans lequel lesdites particules microscopiques (38') ont une taille permettant le passage à travers les pores (50', 52') induits par ladite électroporation.

**4.** Appareil selon l'une quelconque des revendications précédentes, dans lequel lesdites particules microscopiques ont une structure en matrice (38') et les molécules (36) sont encapsulées dans la matrice.

**5.** Appareil selon l'une quelconque des revendications précédentes, dans lequel les particules microscopiques (38) ont une charge électrique.

**6.** Appareil selon l'une quelconque des revendications 1 à 3 ou 5, dans lequel lesdites particules microscopiques (38) ont une membrane formant des microbulles et les molécules (36) sont encapsulées à l'intérieur des microbulles.

**7.** Appareil selon l'une quelconque des revendications précédentes, dans lequel ledit moyen pour appliquer le champ électrique comprend une pluralité d'électrodes étroitement rapprochées (44, 46) appliquées à la surface du tissu (42) et ledit champ est appliqué par impulsions de 10 à plusieurs centaines de volts avec une durée d'impulsion comprise dans la plage allant de 100 μs à 100 ms.

**8.** Appareil selon l'une quelconque des revendications précédentes, dans lequel les particules microscopiques (38) ont une structure telle que les molécules (36) sont encapsulées dans une matrice pour permettre une fonction de temps de libération.

**9.** Appareil selon l'une quelconque des revendications précédentes, dans lequel les électrodes (44, 46) sont des bandes parallèles conductrices montées sur le substrat.

**10.** Appareil selon l'une quelconque des revendications précédentes, comprenant un substrat pour contenir le milieu fluide contenant les particules microscopiques, le substrat étant de préférence spongieux.

FIG. 1

SIGNAL
GENERATOR

FLUID
MEDIUM
SOURCE

FIG. 2

FIG. 3

38    38

40

42

## FIG. 4

PULSE    +

44

46    48

−

+    −    +

ELECTRODES

38

40

ELECTRIC
FIELD
LINES

## FIG. 5

42

**FIG. 6**

**FIG. 7**

**FIG. 8**

**FIG. 9**

FIG. 11

40 44 46 48 42

**FIG. 10**

62 64

**FIG. 12**